# EUROPEAN PATENT SPECIFICATION

(11) **EP 1 492 535 B1**
(45) Date of publication and mention of the grant of the patent: **26.10.2005**
(21) Application number: 03723931.6
(22) Date of filing: 07.04.2003
(51) Int. Cl.: A61K 31/496, C07D 215/56

(54) **ANHYDROUS CRYSTALLINE FORMS I AND II OF 1-CYCLOPROPYL-6-FLUORO-8-METHOXY-7-(3-METHYL-1-PIPERAZINYL) 4-OXO-1, 4-DIHYDROQUINOLINE-3-CARBOXYLIC ACID (GATIFLOXACIN)**
WASSERFREIE KRISTALLINE FORMEN I UND II DER 1-CYCLOPROPYL-6-FLUORO-8-METHOXY-7-(3-METHYL-1-PIPERAZINYL) 4-OXO-1, 4-DIHYDROQUINOLINE-3-CARBONSÄURE (GATIFLOXACIN)
FORMES CRISTALLINES ANHYDRES I ET II DE L'ACIDE 1-CYCLOPROPYL-6-FLUORO-8-METHOXY-7-(3-METHYL-1-PIPERAZINYL) 4-OXO-L, 4-DIHYDROQUINOLEINE-3-CARBOXYLIQUE (GATIFLOXACINE)

(30) Priority: 08.04.2002 IN MA02592002; 12.04.2002 IN MA02852002
(43) Date of publication of application: 05.01.2005
(73) Proprietor: Dr. Reddy's Laboratories Ltd., Hyderabad 500 016, Andhra Pradesh (IN)
(72) Inventor: REDDY, Manne, Satyanarayana, Hyderabad, A.p., 500 016 (IN); RAJU, Chakilam, Naga, Hyderabad, A.P., 500 016 (IN); RAJU, Vetukuri, Venkata, Naga, Kali, vara, Prasada, Hyderabad, A.P., 500 016 (IN); REDDY, Ningam, Srinivas, Hyderbad, A.P., 500 016 (IN); KUMAR, rapolu, Rajesh, Hyderbad, A.P., 500 016 (IN)
(74) Representative: Bates, Philip Ian
(86) International application number: PCT/US2003/010708
(87) International publication number: WO 2003/086402

(56) References cited:
- EP-A- 0 805 156
- US-A- 4 980 470
- US-A- 4 997 943

## Description

The present invention relates to novel anhydrous crystalline Forms of 1-Cyclopropyl-6-fluoro-8-methoxy-7-(3-methyl-1-piperazinyl)-4-oxo 1,4-dihydroquinoline 3-carboxylic acid. The present invention also relates to methods of making these anhydrous Forms of Gatifloxacin.

1-Cyclopropyl-6-fluoro-8-methoxy-7-(3-methyl-1-piperazinyl)-4-oxo 1,4-dihydroquinoline-3-carboxylic acid is generically known as Gatifloxacin and is marketed under brand name "Tequin".

Gatifloxacin can be depicted as Formula (I).

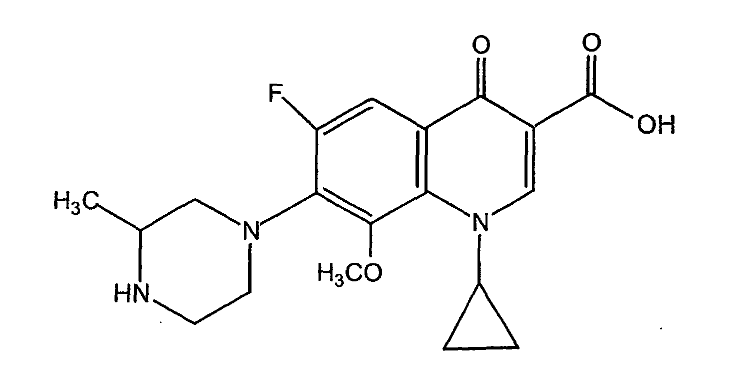

Gatifloxacin and its pharmaceutically acceptable salts are useful as antibiotics.

Quinolone carboxylic acid derivatives constitute a class of extremely potent and orally active broad-spectrum antibacterial agents. Several structural activity relationship (SAR) and quantitative structure activity relationship (QSAR) studies have led to the discovery of an important class of quinolines called fluoroquinolones.

Gatifloxacin of Formula (I) belonging to the class of fluoroquinolones has potent antibacterial activity. Gatifloxacin also has higher selectivity against bacteria from mammalian cells which results in excellent selective toxicity.

Gatifloxacin is preferably administered orally or intravenously. The usual dose of Gatifloxacin is 400mg once daily.

Several references disclose the structure of Gatifloxacin and U.S. Patent 4,980,470, incorporated herein by reference, describes the synthesis of Gatifloxacin hemihydrate. Gatifloxacin hemihydrate is prepared by condensing 1-cyclopropyl-6, 7-difluoro-1, 4-dihydro-4-oxo-8-methoxy quinoline-3-carboxylic acid with 2-methyl piperezine in DMSO, accompanied by chromatographic purification.

U.S. 5,880,283 discloses preparation of the Gatifloxacin sesquihydrate, which involves heating the mixture of 1-Cyclopropyl-6-fluoro-8-methoxy-7-(3-methyl-1-piperazinyl)-4-oxo-1, 4-dihydroquinoline-3-carboxylic acid with water, preferably 3-5 times the weight of reactant, at 80-85°C and subsequent filtration and drying resulting in the sesquihydrate of Gatifloxacin.

Japanese unexamined Patent Publication 63-198664, discloses Gatifloxacin hydrochloride salts. The publications states that the hemihydrate and hydrochloride salt of Gatifloxacin are unstable due to the hygroscopic nature of the drug substance. Problems are encountered due to its poor disintegration and dissolution rate while formulating the tablets.

The present invention is directed to anhydrous crystalline forms of Gatifloxacin, which are non-hygroscopic, crystalline and non-solvated. Generally, the hygroscopic nature is due to the presence of impurities, but the present inventive Forms are non- hygroscopic, which infers the high purity of the compounds. The present inventive Forms are produced in non- solvated form, i.e., the content of residual solvents are well within the limits as per ICH guidelines; hence they are well suited for pharmaceutical formulations.

Another aspect of the present invention is to provide processes for the preparation of the anhydrous crystalline Form I and II of Gatifloxacin, which are cost effective, commercially viable and well suited for industrial scale up.

### BRIEF DESCRIPTION OF THE ACCOMPANYING DRAWING

Fig. 1 is a diagram showing the results of thermogravimetric analysis of the anhydrous crystalline Form I of Gatifloxacin.
Fig. 2 is a diagram showing the results of X-ray diffraction of the anhydrous crystalline Form I of Gatifloxacin.
Fig. 3 is a diagram showing the results of DSC of the anhydrous crystalline Form I of Gatifloxacin.
Fig. 4 is a diagram showing the IR spectrum of the anhydrous crystalline Form I of Gatifloxacin.
Fig. 5 is a diagram showing the results of X-ray diffraction of anhydrous crystalline Form II of Gatifloxacin.
Fig. 6 is a diagram showing the results of DSC of the anhydrous crystalline Form II of Gatifloxacin.
Fig. 7 is a diagram showing the IR spectrum of anhydrous crystalline Form II of Gatifloxacin.

The present invention provides anhydrous crystalline Forms I and II of Gatifloxacin of Formula (1) and processes for the preparation thereof.

The crystalline nature of anhydrous Form I and II of Gatifloxacin is characterized by their respective X-ray diffractograms, Differential Scanning colorimetry thermograms and IR spectrums.

The anhydrous nature of the Forms I and II was characterized by their respective thermogravimetric analysis, and the anhydrous nature of the compounds was also confirmed by calculating the water content present in the compounds by Karl Fischer (KF) method.

The moisture content of the anhydrous Form I of Gatifloxacin ranges from 0.05% to 2.0% by KF and as per thermogravimetric analysis, which is less than the hemihydrate. This confirms the anhydrous nature of the compound. Preferably Form I of Gatifloxacin has a moisture content of 0.4% by KF method.

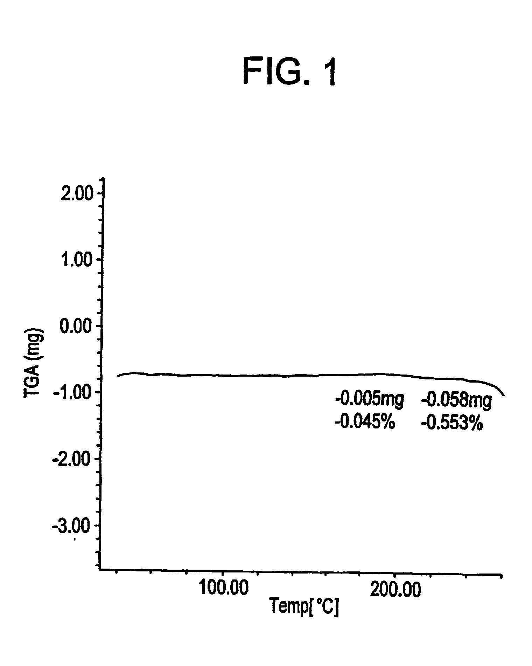

The present invention provides the thermogram of thermo gravimetric analysis of anhydrous Form I of Gatifloxacin substantially as depicted in Figure 1.

The report of thermogravimetric analysis shows a total weight loss of 0.6% at a temperature range of 0-250°C. The result indicates the anhydrous nature of the Form I of Gatifloxacin.

The X-ray powder diffraction pattern of anhydrous crystalline Form I was measured on a Bruker Axs, D8 Advance Powder X-ray Diffractometer with Cu K alpha-1 Radiation source. The samples was scanned between 3 and 45 degrees 2 theta. The anhydrous crystalline Form I of Gatifloxacin has X-ray powder diffraction pattern essentially as shown in the Table-1. The X-ray powder diffraction pattern is expressed in terms of the 2 theta (degrees), and percentage of intensity.

**TABLE 1**

| 2 theta (°) | Intensity (%) |
|---|---|
| 7.763 | 100 |
| 19.722 | 100 |
| 13.615 | 89.3 |
| 25.927 | 71.9 |
| 12.854 | 71.7 |
| 28.65 | 40.9 |
| 20.491 | 35.2 |
| 14.112 | 29.3 |
| 10.196 | 26.8 |
| 23.593 | 15.1 |
| 23.765 | 14.9 |
| 16.333 | 12.2 |
| 27.558 | 10.9 |
| 14.932 | 9.8 |
| 21.456 | 9.7 |
| 30.496 | 8.4 |
| 17.013 | 7.2 |
| 30.872 | 6.7 |
| 31.477 | 5.6 |
| 24.44 | 3.7 |

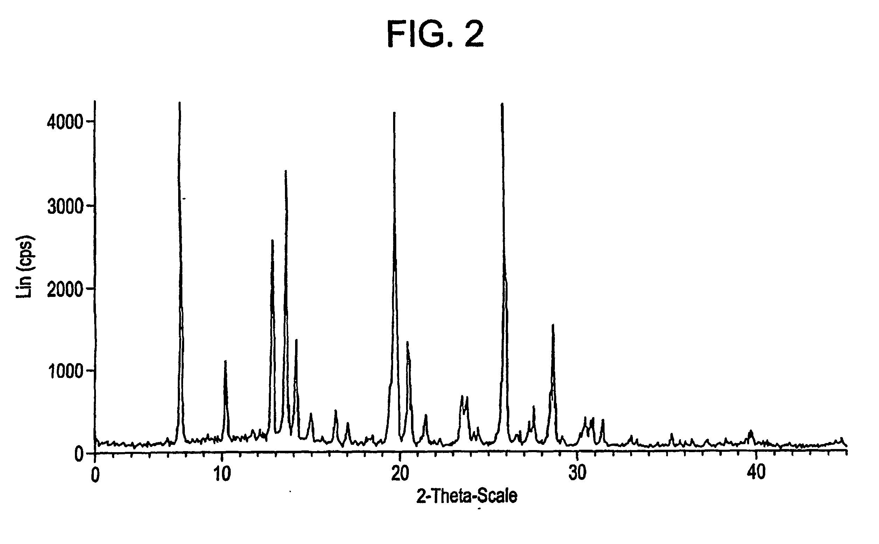

The present invention of anhydrous crystalline Form I of Gatifloxacin is characterized by its X-ray powder diffraction substantially as depicted in Figure 2.

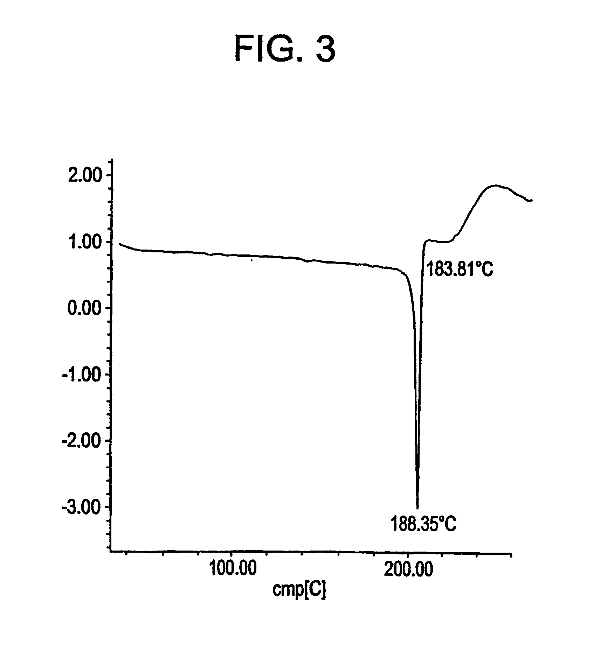

The Differential Scanning Calorimetry thermogram of crystalline Form I exhibits a significant endo peak at 188.35°C and is substantially as depicted in Figure 3.

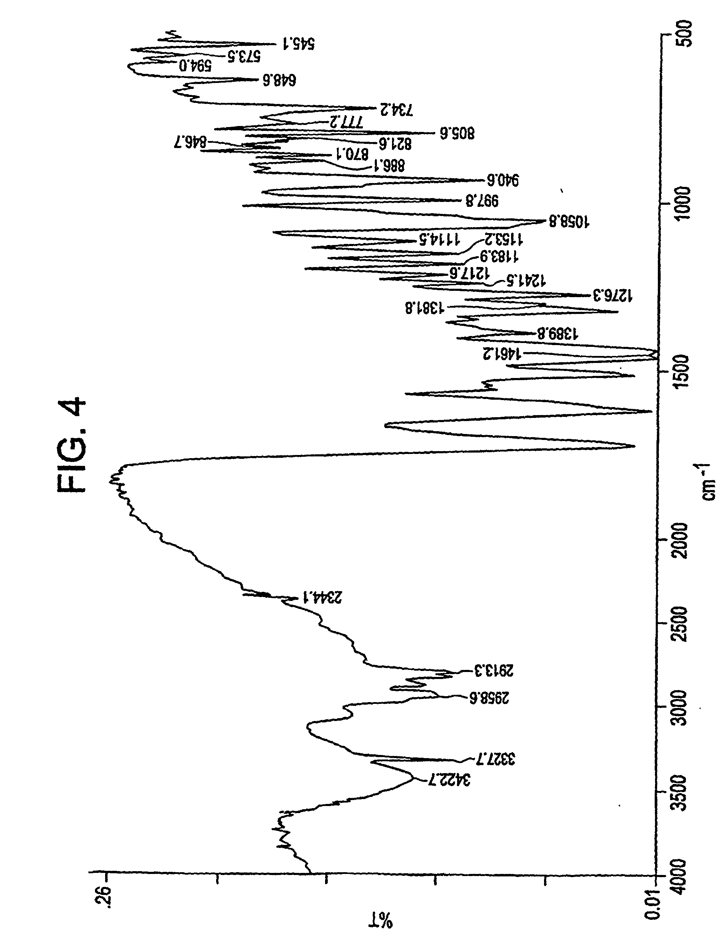

The Infrared data for anhydrous crystalline Form I of Gatifloxacin, was measured by KBr-transmission method and is substantially as depicted in Fig. 4 and shows significant peaks about 3327.7 and 1721.0 cm⁻¹.

Another embodiment of the present invention is to provide a process for the preparation of anhydrous crystalline Form I of Gatifloxacin. Anhydrous crystalline Form I of Gatifloxacin can be prepared by a process which comprises the azeotropic removal of water from a hydrated form of Gatifloxacin at reflux temperature using an aromatic or aliphatic hydrocarbon solvent or a ketone solvent. The process comprises:
i) refluxing azeotropically a hydrated form of Gatifloxacin in water-immiscible aromatic or aliphatic hydrocarbon solvent or ketone solvent;
ii) cooling the reaction mixture of step (i) accompanied by stirring of the mixture until the solid mass crystallizes;
iii) isolating the solid obtained in step (ii) by conventional methods; and
iv) drying the isolated solid of step (iii) at 30-70°;
v) dissolving the solid isolated in step iv) in a linear or branched chain substituted or unsubstituted alkanone solvent at reflux temperature;
vi) cooling to 0-5°C;
vii) filtering and washing with a linear or branched chain substituted or unsubstituted alkanone solvent; and
viii) drying at 30-90°C to obtain anhydrous crystalline Form I of Gatifloxacin.

Preferably the ratio of hydrated Gatifloxacin to solvent is 1:1 to 10 w/w, preferably 1:5 w/v.

As used herein aliphatic hydrocarbon means linear, branched or cyclic hydrocarbon.

Preferably the aromatic or aliphatic hydrocarbon solvent is selected from benzene, toluene, xylene, or cyclohexane. Preferably the ketone is selected from methyl ethyl ketone, methyl isobutyl ketone or methyl tertiary butyl ketone. The preferred solvent is toluene. In step (iii) the solid can be isolated by filtration, decanting, or centrifugation or a combination of these methods.

Preferably in step iv) the solid is dried at 50-60°C.

The ratio of solid to solvent in step v) is the range of 1-40 w/v: 1-40 w/v. Preferably the solvent used in steps v) and vii) is acetone. Preferably the solid in step viii) the solvent is dried at 40-70°C.

The anhydrous crystalline Form II of Gatifloxacin of this invention also has a moisture content from 0.05% to 2.0% by KF and as per thermogravimetric analysis, which is less than the hemihydrate. Preferably, the moisture content is 0.48% by KF.

Anhydrous crystalline polymorph Form II of Gatifloxacin has a moisture content of 0.48% by KF method, which confirms the anhydrous nature of the compound.

The X-ray powder diffraction pattern of anhydrous crystalline polymorph Form II was measured on a Bruker Axs, D8 Advance Powder X-ray Diffractometer with Cu K alpha-1 Radiation source. The sample was scanned between 3 and 45 degrees 2 theta. The anhydrous crystalline polymorph Form II of Gatifloxacin has X-ray powder diffraction pattern essentially as shown in the Table-2. The X-ray powder diffraction pattern is expressed in terms of the 2 theta (degrees), and percentage of intensity.

**TABLE 2**

| 2 theta (°) | Intensity (%) |
|---|---|
| 5.931 | 100 |
| 14.116 | 68.5 |
| 22.509 | 33.7 |
| 21.073 | 31.3 |
| 15.833 | 20.7 |
| 27.571 | 15.9 |
| 27.807 | 15.6 |
| 11.248 | 13.0 |
| 12.426 | 10.2 |
| 23.249 | 9.2 |
| 11.906 | 8.6 |
| 24.287 | 8.0 |
| 29.389 | 6.2 |
| 21.507 | 4.7 |
| 24.943 | 4.3 |
| 17.991 | 3.7 |
| 19.646 | 3.2 |
| 18.667 | 3.0 |
| 39.841 | 2.3 |
| 26.918 | 2.3 |
| 28.504 | 2.2 |
| 16.276 | 2.2 |

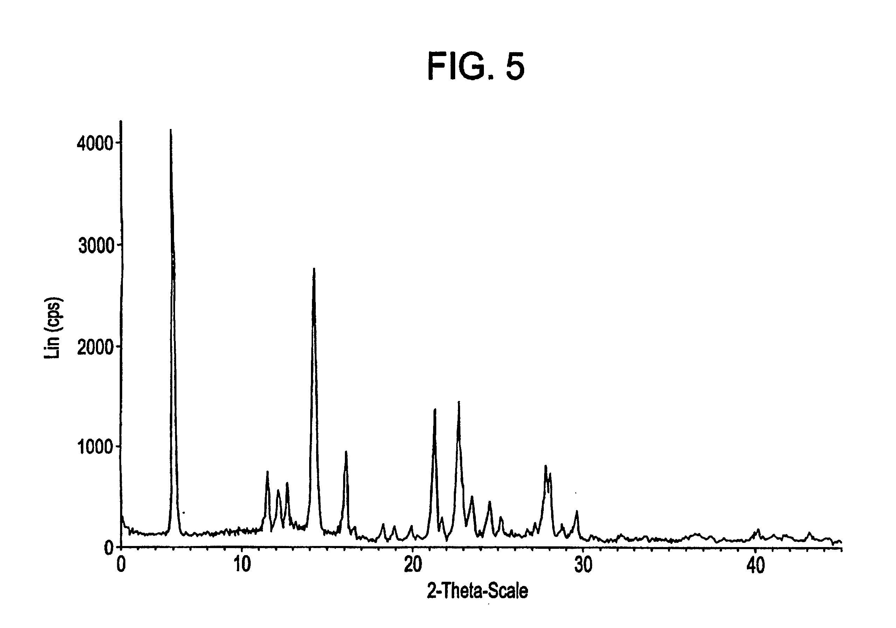

The present invention of anhydrous crystalline polymorph Form-II of Gatifloxacin is characterized by its X-ray powder diffraction pattern substantially as depicted in Figure 5.

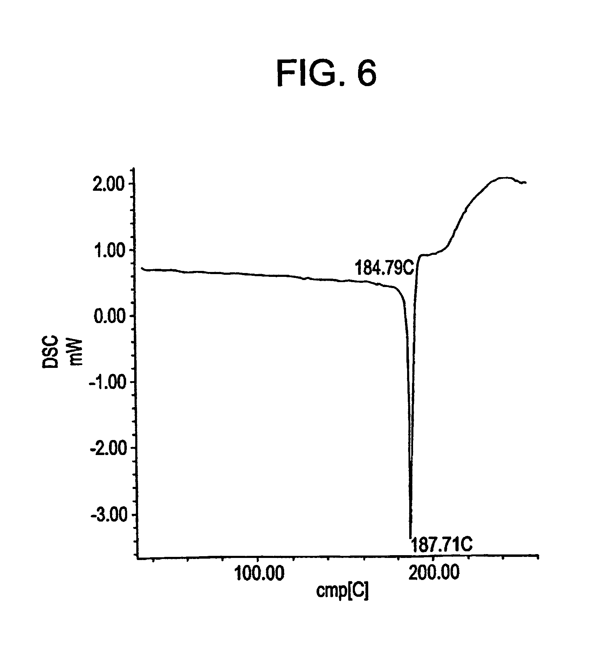

The Differential Scanning Calorimetry thermogram of crystalline Polymorph Form II exhibits a significant endo peak at 187.71 °C and is substantially as depicted in Figure 6.

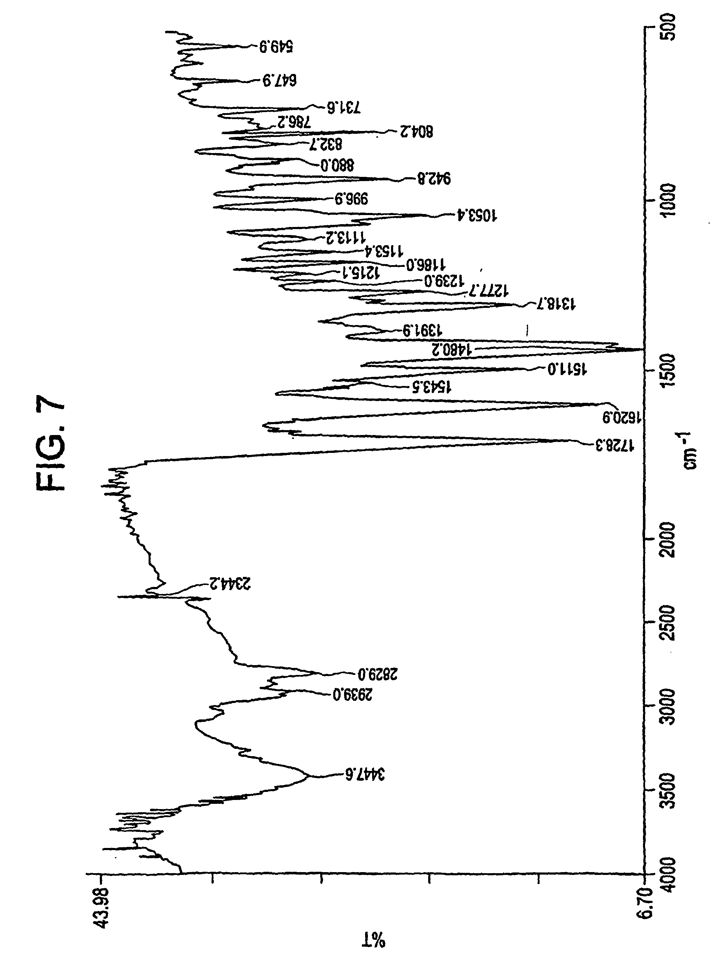

Infrared data for anhydrous crystalline polymorph Form-II of Gatifloxacin, was measured by KBr-transmission method and is substantially as depicted in Fig. 7 with identified significant peaks at about 1620.9 and 1728.3 cm¹.

Another embodiment of the present invention is to provide a process for the preparation of novel anhydrous crystalline Form II of Gatifloxacin. Anhydrous crystalline polymorph Form II of Gatifloxacin can be prepared by a process which comprises the azeotropic removal of water from hydrated form of Gatifloxacin at reflux temperature using an aliphatic hydrocarbon solvent. The process comprises:
i) refluxing azeotropically a hydrated form of Gatifloxacin in a water-immiscible aliphatic hydrocarbon solvent;
ii) cooling the reaction mixture of step (i) accompanied by stirring of the mixture until the solid mass crystallizes;
iii) isolating the solid obtained in step (ii) by conventional methods; and
iv) drying the isolated solid of step (iii) with or without vacuum at 30-70°C to obtain anhydrous crystalline polymorph Form-II of Gatifloxacin.

Preferably the ratio of hydrated Gatifloxacin to solvent is 1:1 to 10 w/w, preferably 1:5 w/v.

As used herein aliphatic hydrocarbon means linear, branched or cyclic hydrocarbon. Preferably, the aliphatic hydrocarbon solvent is cyclohexane.

Preferably, the solid is dried at 40-50°C.

The anhydrous crystalline Forms I and II Gatifloxacin can be prepared from any hydrated Form of Gatifloxacin.

These processes are commercially viable and well suited for industrial scale up.

In step (iii) the solid can be isolated by filtration, decanting or centrifugation or a combination of these methods.

Preferred salts of Form I or II of Gatifloxacin are hydrochloride salts.

The compounds of this invention have antibacterial activity and are useful as antibiotics.

The present invention also envisages pharmaceutical compositions of the anhydrous crystalline Form I and II of 1-Cyclopropyl- 6-fluoro-8-methoxy-7-(3-methyl-1-piperazinyl)-4-oxo-1, 4-dihydro-quinoline-3-carboxylic acid. The compositions may include a physiological acceptable carrier, diluent, excipient, additive, filler, lubricant, binder, stabilizer, solvent or solvate or a mixture thereof.

The pharmaceutical composition may be in a form normally employed, such as tablets, capsules, lozenges, powders, syrups, solutions, suspensions, ointments, dragees and the like, may contain flavourants, sweetners, etc. in suitable solid or liquid carriers or diluents, or in suitable sterile media to form injectable solutions or suspensions. Such compositions typically contain from 1 to 25%, preferably 1 to 15% by weight of active ingredient, the remainder of the composition being one or more of a pharmaceutically acceptable carrier, diluent, excipient, additive, filler, lubricant, binder, stabilizer, solvent or solvate.

The anhydrous crystalline Forms of 1-Cyclopropyl-6-fluoro-8-methoxy-7-(3-methyl-1-piperazinyl)-4-oxo-1, 4-dihydro-quinoline-3-carboxylic acid can be administered to mammals, including man, via either oral, nasal, pulmonary, transdermal or parenteral, rectal, depot, subcutaneous, intravenous, intraurethral, intramuscular, intranasal, ophthalmic solution or an ointment. Administration by the oral route is preferred, being more convenient and avoiding the possible pain and irritation of injection. However, in circumstances where the patient cannot swallow the medication, or absorption following oral administration is impaired, as by disease or other abnormality, it is essential that he drug be administered parenterally. By either route, the dosage is in the range or about 0.01 to about 100 mg/kg body weight of the subject per day or preferably about 0.01 to about 100 mg/kg body weight of the subject per day or preferably about 0.01 to about 30 mg/kg body weight per day administered singly or as a divided dose. However, the optimum dosage for the individual subject being treated will be determined by the person responsible for treatment, generally smaller doses being administered initially and thereafter increments made to determine the most suitable dosage.

Suitable pharmaceutically acceptable carriers include solid fillers or diluents and sterile aqueous or organic solutions. The active ingredient will be present in such pharmaceutical compositions in the amounts sufficient to provide the desired dosage in the range as described above. Thus, for oral administration, the compound can be combined with a suitable solid or liquid carrier or diluent to form capsules, tablets, powders, syrups, solutions, suspensions and the like. The pharmaceutical compositions, may, if desired, contain additional components such a flavourants, sweeteners, excipients and the like. For parenteral administration, the compound can be combined with sterile aqueous or organic media to form injectable solutions or suspensions. For example, solutions in sesame or peanut oil, aqueous solutions of water-soluble pharmaceutically-acceptable acid addition salts or salts with base of the compounds. Aqueous solutions with the active ingredient dissolved in polyhydroxylated castor oil may also be used for injectable solutions. The injectable solutions prepared in this manner can then be administered intravenously, intraperitoneally, subcutaneously, or intramuscularly, with intramuscular administration being preferred in humans.

For nasal administration, the preparation may contain the compound of the present invention dissolved or suspended in a liquid carrier, in particular an aqueous carrier, for aerosol application. The carrier may contain additives such as solubilizing agents, such as propylene glycol, surfactants, absorption enhancers such as lecithin (phosphatidylcholine) or cyclodextrin or preservatives such as parabenes.

Tablets, dragees or capsules having talc and/or a carbohydrate carried binder or the like are particularly suitable for any oral application. Preferably, carriers for tablets, dragees or capsules include lactose, corn starch and/or potato starch. A syrup or elixir can be used in cases where a sweetened vehicle can be employed. An effective amount means that amount of a drug or pharmaceutical agent that will elicit the biological or medical response of a tissue, system, human or animal sought.

The present invention will be explained in more detail by the following non-limiting examples.

### Reference Example

1-cyclo-6, 7-difluoro-1, 4-dihydro-4-oxo-8-methoxy quinolone-3-carboxylic acid (100 grams 0.339 moles) and 2-methyl piperazine (100 grams 1.0 mole) was added to acetonitrile (500 ml) and the reaction mixture was slowly heated to the reflux temperature and stirred until the reaction was substantially complete. Then the solvent was distilled off completely and water (300 ml) was added to the residual mass. The reaction mass was cooled to the temperature of 40-50°C. The pH was adjusted towards acidic with acetic acid (250 ml). The mass was filtered off and then pH of the filtrate was further adjusted to 6.0-8.0 with ammonia. The reaction mixture was cooled to a temperature of 10-15°C and stirred for 30-45 minutes. Thus, the obtained solid was successively recrystallized to afford Gatifloxacin; which had a moisture content ranging from 2.5 to 50.0%.

### EXAMPLE 1

### Preparation Of Anhydrous Crystalline Form I Of Gatifloxacin

Gatifloxacin hydrate 53 grams (obtained as per reference example) was suspended in toluene (250 ml) and heated to reflux to the temperature of 100-110°C. Water was azeotropically removed, accompanied by cooling of the reaction mixture to 10-15°C under stirring for 30-60 minutes to crystallize the solid mass. The crystallized solid mass was filtered, washed with toluene (50 ml) and dried at 50-60°C to constant weight. The obtained product is dissolved in acetone (20 times to solid quantity) at reflux 50-60° C and distill off 15 times of solvent to the initial volume (solid solvent ratio is 1:5 prior to isolation) and reaction mass is cooled to 0-5° and filtered, washed with Acetone (0.5-1.0 times to the input quantity). Finally product is dried at 50-60°C until constant weight is obtained.
(Weight: 38 grams, 40%, M.C. by KF is 0.40% and Purity: 99.83%).

### EXAMPLE 2

### Preparation Of Anhydrous Crystalline Polymorph Form II Of Gatifloxacin

Gatifloxacin hydrate (10 grams, 0.026 moles, prepared as per reference example) was suspended in cyclohexane (50 ml) and heated to reflux to the temperature of 70-80 °C. Water was azeotropically removed, accompanied by cooling of the reaction mixture to 0-10°C under stirring for 30-60 minutes to crystallize the solid mass. The crystallized solid mass was filtered, washed with cyclohexane (10 ml) and dried under reduced pressure at a temperature of 40-50°C to constant weight.
(Weight: 9 grams, 90%, M.C. by KF is 0.48% and Purity: 99.58%).

### Detailed Description Of The Accompanying Drawings

Fig. 1 is characteristic thermogram of thermal gravimetric analysis of anhydrous crystalline form I of Gatifloxacin, shows a total weight loss of 0.60% (w/w) at a temperature range of 0-250°C indicates the anhydrous nature of the inventive substance of crystalline Form I of Gatifloxacin.
Vertical axis: weight of the compound (in mg); Horizontal axis: Temperature (in °C). Vertical axis: Intensity (CPS); Horizontal axis: 2 Theta (degrees).
Scan speed: Time/step: 0.40 secs.
Sampling time: Scan Time: 14:0.40 min
Scan Mode: Continuous
Reflection: Geometry is reflection (nor transmission)
Scan Type: Locked coupled.

Fig: 2 is characteristic X-ray powder diffraction pattern of the novel anhydrous crystalline Form I of Gatifloxacin.
Vertical axis: Intensity (CPS); Horizontal axis: 2 Theta (degrees).

The significant 2 theta values (in degrees) obtained are 7.763, 10.196, 12.854, 13.615, 14.1112, 14.932, 16.333, 17.013, 19.722, 20.491, 21.456, 23.593, 23.765, 24.44, 25.927, 27.558, 28.65, 30.496, 30.872, and 31.477.

Fig: 3 is characteristic Differential Scanning Calorimetric thermogram of anhydrous crystalline Form I of Gatifloxacin. The Differential Scanning Calorimetric thermogram exhibits a significant endo peak at 188.35°C.
Vertical axis: Temperature (in °C); Horizontal axis: Signal (in mV), Heating rate 5 °/minute.

Fig: 4 is characteristic Infra Red spectrum of anhydrous crystalline Form I of Gatifloxacin with identified significant peaks at about 3327.7 and 1721.0 cm⁻¹.
Vertical axis: Wave length (in Cm⁻¹); Horizontal axis: Transmission (in %) KBr.

Fig: 5 is characteristic X-ray powder diffraction pattern of the anhydrous crystalline polymorph Form-II of Gatifloxacin.
Vertical axis: Intensity (CPS); Horizontal axis: 2 Theta (degrees).
Scan speed: Time/step: 0.40 secs.
Sampling time: Scan Time: 14:0.40 min
Scan Mode: Continuous
Reflection: Geometry is reflection (nor transmission)
Scan Type: Locked coupled.

The significant 2 theta values (in degrees) obtained are 5.931, 11.248, 11.906, 12.426, 14.116, 15.833, 16.276, 17.991, 18.667, 19.646, 21.073, 21.507, 22.509, 23.249, 24.287, 24.943, 26.918, 27.571, 27.807, 28.504, 29.389, and 39.841 degrees two theta.

Fig. 6 is characteristic Differential Scanning Calorimetric thermogram of novel anhydrous crystalline polymorph Form II of Gatifloxacin. The Differential Scanning Calorimetric thermogram exhibits a significant endo peak at 187.71 °C.
Vertical axis: Temperature (in °C); Horizontal axis: Signal (in mV), in Heating rate 5°/minute.

Fig. 7 is characteristic Infra Red spectrum of anhydrous crystalline polymorph Form II of Gatifloxacin with identified significant peaks at about 1620.9 and 1728.3 cm⁻¹.
Vertical axis: Wave length (in Cm⁻¹); Horizontal axis: Transmission (in %) KBr.

## Claims

1. An anhydrous crystalline Form I of gatifloxacin, 1-Cyclopropyl-6-fluoro-8-methoxy-7-(3-methyl-1-piperazinyl)-4-oxo-1,4-dihydroquinoline-3-carboxylic acid.

2. An anhydrous crystalline Form I of gatifloxacin according to claim 1, having a differential scanning calorimetry endotherm at 188.35°C.

3. An anhydrous crystalline Form I of gatifloxacin according to claim 1 or 2, having an X-ray powder diffraction pattern with peaks at 7.763, 10.196, 12.854, 13.615, 14.112, 14.932, 16.333, 17.013, 19.722, 20.491, 21.456, 23.593, 23.765, 24.44, 25.927, 27.558, 28.65, 30.496, 30.872, and 31.477 degrees 2θ.

4. An anhydrous crystalline Form I of Gatifloxacin according to any preceding claim, having an infrared absorption spectrum comprising peaks at 3327.7 and 1721.0 cm⁻¹.

5. A process for preparing an anhydrous crystalline Form I of gatifloxacin according to any preceding claim, comprising the steps of:
i) refluxing a hydrated gatifloxacin in a water-immiscible aromatic or aliphatic hydrocarbon, or ketone, solvent to azeotropically remove water;
ii) cooling with stirring to obtain a solid;
iii) dissolving the solid in a linear or branched chain substituted or unsubstituted alkanone solvent at reflux temperatures;
iv) cooling to 0-5°C;
v) separating a solid and washing with a linear or branched chain substituted or unsubstituted alkanone solvent; and
vi) drying at 30-90°C to obtain anhydrous crystalline Form I of gatifloxacin.

6. A process according to claim 5, wherein the aromatic or aliphatic hydrocarbon solvent is selected from benzene, toluene, xylene, or cyclohexane.

7. A process according to claim 5, wherein the ketone solvent is selected from methyl ethyl ketone, methyl isobutyl ketone or methyl tertiary-butyl ketone.

8. An anhydrous crystalline Form II of gatifloxacin, 1-Cyclopropyl-6-fluoro-8-methoxy-7-(3-methyl-1-piperazinyl)-4-oxo-1,4-dihydroquinoline-3-carboxylic acid.

9. An anhydrous crystalline Form II of gatifloxacin according to claim 8 having a differential scanning calorimetry endotherm at 187.71°C.

10. An anhydrous crystalline Form II of gatifloxacin according to claim 8 or 9, having an X-ray powder diffraction pattern with peaks at 5.931, 11.248, 11.906, 12.426, 14.116, 15.833, 16.276, 17.991, 18.667, 19.646, 21.073, 21.507, 22.509, 23.249, 24.287, 24.943, 26.918, 27.571, 27.807, 28.504, 29-389, and 39.841 degrees 2θ.

11. An anhydrous crystalline Form II of gatifloxacin according to any of claims 8, 9 or 10, having infrared absorption peaks at 1620.9 and 1728.3 cm⁻¹.

12. A process for preparing an anhydrous crystalline Form II of gatifloxacin according to any of claims 8 to 11, comprising the steps of:
i. refluxing a hydrated gatifloxacin in a water-immiscible aliphatic hydrocarbon solvent to azeotropically remove water;
ii. cooling with stirring to obtain a solid;
iii. isolating the solid and drying with or without vacuum at 30-70°C, to obtain anhydrous crystalline Form II of gatifloxacin.

13. A process according to claim 12, wherein the aliphatic hydrocarbon solvent is cyclohexane.

## Patentansprüche

1. Wasserfreie, kristalline Form I von Gatifloxacin, 1-cyclopropyl-6-fluor-8-methoxy-7-(3-methyl-1-piperazinyl)-4-oxo-1,4-dihydrochinolin-3-carbonsäure.

2. Wasserfreie, kristalline Form I von Gatifloxacin nach Anspruch 1, mit einem endothermen Höchstwert gemäß Differentialscanningkalorimetrie bei 188,35°C.

3. Wasserfreie, kristalline Form I von Gatifloxacin nach Anspruch 1 oder 2, mit einem Pulverröntgenbeugungsdiagramm mit Peaks bei 7,763, 10,196, 12,854, 13,615, 14,112, 14,932, 16,333, 17,013, 19,722, 20,491, 21,456, 23,593, 23,765, 24,44, 25,927, 27,558, 28,65, 30,496, 30,872 und 31,477 Grad 2θ.

4. Wasserfreie, kristalline Form I von Gatifloxacin nach einem der vorherigen Ansprüche, mit einem Infrarot-Absorptionsspektrum mit Peaks bei 3327,7 und 1721,0 cm⁻¹.

5. Verfahren zur Herstellung einer wasserfreien, kristallinen Form I von Gatifloxacin nach einem der vorherigen Ansprüche, umfassend die folgenden Schritte:
i) Rückflusserhitzen eines hydratisierten Gatifloxacins in einem mit Wasser nicht mischbaren, aromatischen oder aliphatischen Kohlenwasserstoff- oder Ketonlösungsmittel, um Wasser azeotrop zu entfernen;
ii) Kühlen unter Rühren, um einen Feststoff zu erhalten;
iii) Lösen des Feststoffes in einem linearen oder verzweigtkettigen, substituierten oder nicht substituierten Alkanonlösungsmittel bei Rückflusstemperaturen;
iv) Kühlen auf 0 bis 5°C;
v) Trennen eines Feststoffs und Waschen mit einem linearen oder verzweigtkettigen, substituierten oder nicht substituierten Alkanonlösungsmittel; und
vi) Trocknen bei 30 bis 90°C, um die wasserfreie, kristalline Form I von Gatifloxacin zu erhalten.

6. Verfahren nach Anspruch 5, wobei das aromatische oder aliphatische Kohlenwasserstofflösungsmittel ausgewählt wird aus Benzol, Toluol, Xylol oder Cyclohexan.

7. Verfahren nach Anspruch 5, wobei das Ketonlösungsmittel ausgewählt wird aus Methylethylketon, Methylisobutylketon oder Methyltertiar-Butylketon.

8. Wasserfreie, kristalline Form II von Gatifloxacin, 1-Cyclopropyl-6-fluor-8-methoxy-7-(3-methyl-1-piperazinyl)-4-oxo-1,4-dihydrochinolin-3-carbonsäure.

9. Wasserfreie, kristalline Form II von Gatifloxacin nach Anspruch 8, mit einem endothermen Höchstwert gemäß Differentialscanningkalorimetrie bei 187,71°C.

10. wasserfreie, kristalline Form II von Gatifloxacin nach Anspruch 8 oder 9, mit einem Pulverröntgenbeugungsdiagramm mit Peaks bei 5,931, 11,248, 11,906, 12,426, 14,116, 15,833, 16,276, 17,991, 18,667, 19,646, 21,073, 21,507, 22,509, 23,249, 24,287, 24,943, 26,918, 27,571, 27,807, 28,504, 29,389 und 39,841 Grad 2θ.

11. Wasserfreie, kristalline Form II von Gatifloxacin nach einem der Ansprüche 8, 9 oder 10, mit Infrarotabsorptionspeaks bei 1620, 9 und 1728,3 cm⁻¹.

12. Verfahren zur Herstellung einer wasserfreien, kristallinen Form II von Gatifloxacin nach einem der Ansprüche 8 bis 11, das die folgenden Schritte umfasst:
i) Rückflusserhitzen eines hydratisierten Gatifloxacins in einem mit Wasser nicht mischbaren, aliphatischen Kohlenwasserstofflösungsmittel, um Wasser azeotrop zu entfernen;
ii) Kühlen unter Rühren, um einen Feststoff zu erhalten;
iii) Isolieren des Feststoffes und Trocknen mit oder ohne Vakuum bei 30 bis 70°C, um die wasserfreie, kristalline Form II von Gatifloxacin zu erhalten.

13. Verfahren nach Anspruch 12, wobei das aliphatische Kohlenwasserstofflösungsmittel Cyclohexan ist.

## Revendications

1. Forme I cristalline anhydre de gatifloxacine, acide 1-cyclopropyl-6-fluoro-8-méthoxy-7-(3-méthyl-1-pipérazinyl)-4-oxo-1,4-dihydroquinoline-3-carboxylique.

2. Forme 1 cristalline anhydre de gatifloxacine selon la revendication 1, ayant un endotherme d'analyse calorimétrique différentielle à 188,35°C.

3. Forme I cristalline anhydre de gatifloxacine selon la revendication 1 ou 2, ayant un réseau de diffraction des rayons X sur poudre avec des pics à 7,763, 10,196, 12,854, 13,615, 14,112, 14,932, 16,333, 17,013, 19,722, 20,491, 21,456, 23,593, 23,765, 24,44, 25,927, 27,558, 28,65, 30,496, 30,872 et 31,477 2θ en degrés.

4. Forme 1 cristalline anhydre de gatifloxacine selon l'une quelconque des revendications précédentes, ayant un spectre d'absorption infrarouge comprenant des pics à 3327,7 et à 1721,0 cm⁻¹.

5. Procédé de préparation d'une forme I cristalline anhydre de gatifloxacine selon l'une quelconque des revendications précédentes, comprenant les étapes consistant à :
i) porter à reflux une gatifloxacine hydratée dans un solvant d'hydrocarbure aromatique ou aliphatique ou de cétone, non miscible avec l'eau, afin de retirer l'eau de manière azéotrope;
ii) refroidir en agitant pour obtenir un solide;
iii) dissoudre le solide dans un solvant d'alcanone à chaîne linéaire ou ramifié substituée ou non substituée à des températures de reflux;
iv) refroidir à 0-5°C;
v) séparer un solide et laver avec un solvant d'alcanone à chaîne linéaire ou ramifié substituée ou non substituée; et
vi) sécher à 30-90°C pour obtenir une forme I cristalline anhydre de gatifloxacine.

6. Procédé selon la revendication 5, dans lequel le solvant d'hydrocarbure aromatique ou aliphatique est sélectionné parmi le benzène, le toluène, le xylène ou le cyclohexane.

7. Procédé selon la revendication 5, dans lequel le solvant de cétone est sélectionné parmi la méthyl-éthyl-cétone, la méthyl-isobutyl-cétone ou la méthyl-butyl-tertiaire-cétone.

8. Forme II cristalline anhydre de gatifloxacine, acide 1-cyclopropyl-6-fluoro-8-méthoxy-7-(3-méthyl-1-pipérazinyl)-4-oxo-1,4-dihydroquinoline-3-carboxylique.

9. Forme II cristalline anhydre de gatifloxacine selon la revendication 8, ayant un endotherme d'analyse calorimétrique différentielle à 187,71°C.

10. Forme II cristalline anhydre de gatifloxacine selon la revendication 8 ou 9, ayant un réseau de diffraction des rayons X sur poudre avec des pics à 5,931, 11,248, 11,906, 12,426, 14,116, 15,833, 16,276, 17,991, 18,667, 19,646, 21,073, 21,507, 22,509, 23,249, 24,287, 24,943, 26,918, 27,571, 27,807, 28,504, 29,389 et 39,841 2θ en degrés.

11. Forme II cristalline anhydre de gatifloxacine selon l'une quelconque des revendications 8, 9 ou 10, ayant des pics d'absorption infrarouge à 1620,9 et à 1728,3 cm⁻¹.

12. Procédé de préparation d'une forme II cristalline anhydre de gatifloxacine selon l'une quelconque des revendications 8 à 11, comprenant les étapes consistant à :
i. porter à reflux une gatifloxacine hydratée dans un solvant d'hydrocarbure aliphatique, non miscible avec l'eau, afin de retirer l'eau de manière azéotrope;
ii) refroidir en agitant pour obtenir un solide;
iii) dissoudre le solide et sécher avec ou sans vide à 30-70°C, pour obtenir une forme Il cristalline anhydre de gatifloxacine.

13. Procédé selon la revendication 12, dans lequel le solvant d'hydrocarbure aliphatique est du cyclohexane.
